# FASCICULE DE BREVET EUROPEEN

(11) **EP 0 838 449 B1**
(45) Date de publication et mention de la délivrance du brevet: **31.10.2001**
(21) Numéro de dépôt: 97402485.3
(22) Date de dépôt: 20.10.1997
(51) Int. Cl.: C07C 11/09, C07C 11/06, C07C 7/163, C07C 5/25, C07C 6/04, B01J 23/44

(54) **Nouveau procédé de production d'isobutène et de propylène à partir de coupes d'hydrocarbures à quatre atomes de carbone**
Verfahren zur Herstellung von Isobuten und Propen aus Kohlenwasserstofffraktionen mit vier Kohlenstoffatomen
Process for the production of isobutene and propene from hydrocarbon fractions with four carbon atoms

(30) Priorité: 28.10.1996 FR 9613298
(43) Date de publication de la demande: 29.04.1998
(73) Titulaire: INSTITUT FRANCAIS DU PETROLE, 92852 Rueil-Malmaison Cedex (FR)
(72) Inventeur: Chodorge, Jean-Alain, 92160 Antony (FR); Cosyns, Jean, 78580 Maule (FR); Commereuc, Dominique, 92190 Meudon (FR)

(56) Documents cités:
- EP-A- 0 639 549
- EP-A- 0 742 234
- BE-A- 735 727
- FR-A- 1 493 983
- US-A- 5 120 894

## Description

L'invention concerne un procédé de production d'isobutène et de propylène à partir d'une coupe C₄.

Le vapocraquage de charges constituées par des coupes paraffiniques légères fournit l'éthylène et le propylène nécessaires à la pétrochimie. Il fournit également un certain nombre d'autres produits plus lourds, et en particulier une coupe d'hydrocarbures en C₄ qui contient principalement du butadiène-1,3, de l'isobutène, des n-butènes et des butanes, accompagnés de traces d'hydrocarbures acétyléniques.

Le craquage catalytique de charges d'hydrocarbures lourds, fournit, à côté des fractions essence et gazole qui sont les produits principaux, des produits plus légers, parmi lesquels une coupe d'hydrocarbures en C₄ qui contient principalement de l'isobutane, de l'isobutène, des n-butènes et des butanes, accompagnés de faibles quantités de butadiène-1,3 et d'hydrocarbures acétyléniques.

Jusqu'à récemment, seuls le butadiène-1,3 et l'isobutène trouvaient un usage dans l'industrie des polymères, en particulier dans l'industrie des pneumatiques pour le premier. L'augmentation de la longévité des pneumatiques et une relative stagnation de la demande font qu'il existe maintenant des surplus de butadiène qui ne sont pas, ou mal, utilisés. Au contraire, il y a un regain d'intérêt pour l'isobutène qui peut être utilisé par exemple pour la synthèse d'éthers à usage d'additifs dans les carburants automobiles ou comme monomère dans la synthèse de polyisobutène.

Le brevet BE-A-0735.727 décrit un procédé d'isomérisation des oléfines accompagnée simultanément d'une hydrogénation des composés dioléfiniques et acétyléniques pour obtenir un effluent enrichi en butène-2.

Egalement, dans le brevet FR-A-1 493 983, il est décrit un procédé pour l'isomérisation du butène-1 en butène-2 en présence d'isobutène à l'aide d'un catalyseur supporté au nickel et sulfuré.

Le Brevet US-5,120,894 enseigne un procédé de traitement d'une coupe C4 incluant une isomérisation du butène-1 en butène-2 suivie d'une métathèse de la totalité de l'effluent non purifié.

La particularité de ce procédé de métathèse est d'être appliqué à des charges non purifiées. Ainsi, de façon préférée, la charge isomérisée traverse ensuite un lit de catalyseur de métathèse mélangé à un catalyseur d'isomérisation. L'isomérisation est alors également recherchée simultanément au déroulement de la réaction de métathèse.

La présente invention propose un procédé de traitement d'une coupe d'hydrocarbures en C₄ contenant principalement de l'isobutène, des n-butènes, des butanes, et du butadiène-1,3 en quantité variable, qui inclut la séparation de l'isobutène par distillation, et qui permet de transformer le butadiène-1,3 et les n-butènes en propylène utilisable par exemple pour la polymérisation.

Les proportions relatives d'éthylène et de propylène produits dans une opération de vapocraquage peuvent être modulées dans une certaine mesure en changeant la nature de la charge et en modifiant les conditions opératoires (la sévérité) du craquage. Cependant, le mode de fonctionnement orienté vers une proportion plus grande de propylène entraîne inévitablement une baisse du rendement en éthylène et une production plus importante de coupe C₄ et de fraction essence.

Un autre but de la présente invention est d'augmenter la production de propylène tout en maintenant un haut rendement en éthylène grâce au traitement de la coupe d'hydrocarbures en C₄ et donc sans être obligé de baisser la sévérité du vapocraqueur.

Le procédé objet de l'invention est plus précisément un procédé de conversion d'une coupe C₄ oléfinique en isobutène et en propylène, ladite coupe contenant notamment des dioléfines, du butène-1, du butène-2, de l'isobutène et des impuretés acétyléniques, ledit procédé comportant les étapes suivantes se déroulant successivement :
1) hydrogénation sélective des dioléfines et des impuretés acétyléniques avec isomérisation du butène-1 en butène-2 de façon à obtenir un effluent contenant majoritairement du butène-2 et de l'isobutène, et ne contenant pratiquement pas de dioléfines ni de composés acétyléniques, de préférence par passage de ladite coupe en phase liquide sur un catalyseur comprenant au moins un métal choisi dans le groupe formé par le nickel, le palladium et le platine, déposé sur un support, à une température de 20-200 °C, une pression de 0,1-5 MPa, une vitesse spatiale de 0,5-10 h⁻¹, avec un rapport H₂ / dioléfines (molaire) de 0,5 à 5 et de préférence de 1 à 3,
2) séparation par distillation d'une coupe de tête contenant majoritairement l'isobutène et le butène-1 non converti dans la première étape, et d'une coupe de pied contenant essentiellement le butène-2 et le butane, au plus 1 % en poids d'isobutène et au plus 1 % en poids de butène-1,
3) métathèse de la coupe butène-2 issue de l'étape précédente avec de l'éthylène, en présence d'un catalyseur comportant au moins un oxyde de rhénium déposé sur un support, à une température comprise entre 0 et 100 °C, et à une pression au moins égale à la tension de vapeur du mélange réactionnel à la température de réaction, de façon à obtenir un effluent contenant du propylène, la métathèse étant suivie d'une séparation du propylène.

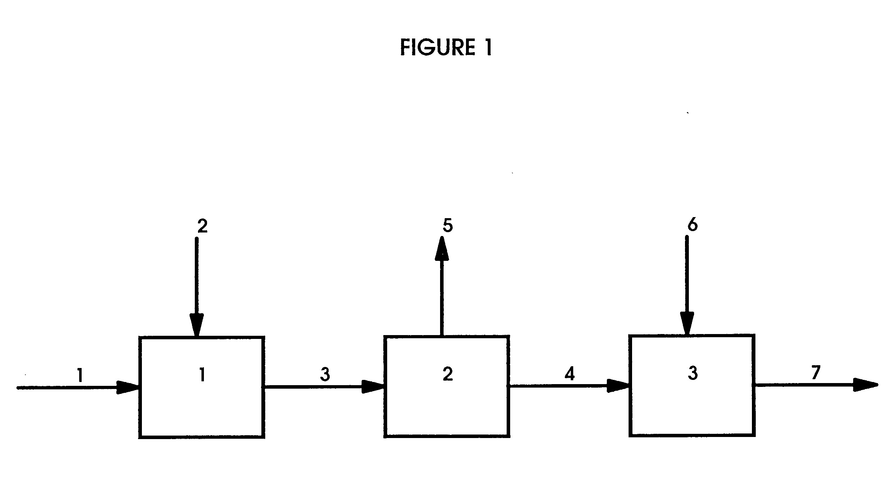

On décrira plus en détail le procédé selon l'invention schématisé sur la figure 1, à partir d'une coupe d'hydrocarbures en C4 entrant par un conduit 1, qui contient principalement de l'isobutène, des n-butènes, des butanes, ainsi que du butadiène en quantité variable. La coupe C₄ est soumise à une succession de traitements regroupés dans les étapes suivantes, pour produire de l'isobutène et du propylène :
- hydrogénation sélective des hydrocarbures acétyléniques et du butadiène avec isomérisation du butène-1 en butène-2,
- séparation de l'isobutène et du butène-1 d'avec le butène-2,
- métathèse du butène-2 en présence d'éthylène (éthènolyse) produisant du propylène.

La succession des traitements retenue dans le procédé selon l'invention présente de nombreux avantages. Les composés les plus réactifs de la coupe, à savoir le butadiène-1,3 par exemple, en quantité variable, ainsi que les traces d'hydrocarbures acétyléniques sont transformés dès la première étape, et ne seront donc pas la cause de réactions parasites dans les étapes suivantes. Par ailleurs l'hydrogénation sélective des dioléfines (butadiène-1,3 et -1,2) en butènes et l'hydroisomérisation du butène-1 permet d'augmenter considérablement la concentration en butène-2 dans la coupe, ce qui favorise d'autant un rendement élevé en propylène dans l'étape de métathèse.

Le fractionnement de la coupe issue de l'hydroisomérisation, en isobutène et butène-1 d'une part, et en butène-2 d'autre part, permet de valoriser de façon diversifiée l'isobutène sans introduire de polluants dans la fraction contenant le butène-2 qui est ensuite soumise à la métathèse. Il conduit également à l'emploi d'un catalyseur de métathèse ayant une action isomérisante faible, voire pratiquement nulle. De plus, si par exemple l'isobutène est transformé en méthyl-tertiobutyléther par réaction avec le méthanol, le butène-2 ne sera pas souillé par des sous-produits oxygénés comme il est habituel lorsque cette réaction est effectuée directement sur la coupe hydroisomérisée. Il en est de même si l'isobutène est transformé par polymérisation, qui est une voie préférée de valorisation de l'isobutène.

De plus, dans l'étape suivante de métathèse, la faible teneur en butène-1 dans la fraction riche en butène-2 permet d'obtenir une sélectivité en propylène voisine de 100 %. En effet, le butène-1 réagit avec le butène-2 pour donner du propylène et des pentènes, et il réagit sur lui-même pour donner des hexènes. Pentènes et hexènes sont des sous-produits de faible valeur dont il faut se débarrasser de manière coûteuse. Le procédé permet donc une augmentation appréciable du rendement en propylène, et facilite le recyclage du butène-2 au réacteur de métathèse, puisqu'il y a peu de pentènes et d'hexènes à éliminer.

L'invention concerne également une installation (illustrée figure 1) pour mettre en oeuvre le procédé décrit ci-dessus et qui comporte successivement :
- une zone 1 d'hydrogénation sélective avec isomérisation du butène-1 en butène-2, ladite zone comportant au moins un moyen 1 pour l'introduction de la coupe à convertir, au moins un moyen 3 pour la sortie de l'effluent et au moins un moyen 2 pour l'introduction de l'hydrogène, ladite zone comportant également au moins un lit de catalyseur qui comprend de préférence au moins un métal choisi dans le groupe formé par le nickel, le palladium et le platine, déposé sur un support,
- une zone 2 de séparation, comportant au moins un moyen 3 pour l'introduction de l'effluent issu de la zone 1, au moins un moyen 5 pour la sortie de l'isobutène et du butène-1, au moins un moyen 4 pour la sortie du butène-2 et du n-butane,
- une zone 3 de métathèse contenant au moins un catalyseur de préférence à base d'oxyde de rhénium déposé sur un support, et comprenant au moins un moyen 4 d'introduction de l'effluent issu de la zone 2, au moins un moyen 6 d'introduction de l'éthylène et au moins un moyen 7 pour la sortie du propylène.

De façon particulièrement intéressante, la coupe C₄ provient d'une zone de vapocraquage en amont, le moyen d'introduction de la coupe à convertir dans la zone 1 étant relié à ladite zone de vapocraquage, et le moyen d'introduction de l'éthylène dans la zone 4 étant relié à ladite zone de vapocraquage.

L'invention sera décrite à partir de la figure 1.

L'objet principal de la première étape est de transformer le butadiène et les n-butènes en butène-2. En effet, le butène-2 est la source du propylène qui est produit dans la dernière étape de métathèse en présence d'éthylène. Il est donc souhaitable de maximiser le rendement en butène-2, c'est-à-dire de se rapprocher autant que possible de la proportion autorisée par la thermodynamique. Le deuxième objet de cette étape est d'éliminer les traces d'hydrocarbures acétyléniques toujours présentes dans ces coupes et qui sont des poisons ou des polluants pour les étapes ultérieures.

Dans cette première étape (zone 1), on réalise donc simultanément les réactions suivantes , en présence d'hydrogène amené par un conduit 2 :
- hydrogénation sélective du butadiène en un mélange de n-butènes à l'équilibre thermodynamique,
- isomérisation du butène-1 en butène-2, de manière à obtenir une répartition proche de l'équilibre thermodynamique,
- hydrogénation sélective des traces d'hydrocarbures acétyléniques en butènes.

Ces réactions peuvent être réalisées au moyen de divers catalyseurs spécifiques comprenant un ou plusieurs métaux, par exemple du groupe 10 de la classification périodique (Ni, Pd, Pt), déposés sur un support. On met en oeuvre de préférence un catalyseur qui comprend au moins un composé de palladium fixé sur un support minéral réfractaire, par exemple sur une alumine. La teneur en palladium sur le support peut être comprise entre 0,01 et 5 % en poids, de préférence entre 0,05 et 1 % en poids. Divers modes de prétraitement connus de l'homme de l'art peuvent éventuellement être appliqués à ces catalyseurs pour améliorer la sélectivité dans l'hydrogénation du butadiène en butènes aux dépens de l'hydrogénation totale en butane qu'il faut éviter. De préférence le catalyseur contient 0,05-10 % en poids de soufre. De façon avantageuse, on utilise un catalyseur constitué par du palladium déposé sur de l'alumine, et du soufre.

Le catalyseur peut avantageusement être mis en oeuvre selon le procédé décrit dans le brevet FR-93/09529, c'est-à-dire que le catalyseur a été traité, avant son chargement dans le réacteur d'hydrogénation, par au moins un composé soufré dilué dans un solvant, puis le catalyseur obtenu contenant 0,05 à 10 % de soufre (en poids) est chargé dans le réacteur et activé sous atmosphère neutre ou réductrice à une température comprise entre 20 et 300 °C, une pression comprise entre 0,1 et 5 MPa et une VVH comprise entre 50 et 600 h⁻¹, et la charge est mise au contact dudit catalyseur activé.

La mise en oeuvre du catalyseur, de préférence au palladium, n'est pas critique, mais on préfère généralement utiliser au moins un réacteur à flux descendant à travers un lit fixe de catalyseur. Lorsque la proportion de butadiène dans la coupe est importante, ce qui est le cas par exemple d'une coupe de vapocraquage lorsqu'on ne souhaite pas en extraire le butadiène pour des usages spécifiques, il peut être avantageux d'effectuer la transformation dans deux réacteurs en série afin de mieux contrôler la sélectivité de l'hydrogénation. Le second réacteur peut être à flux ascendant et avoir un rôle de finition.

La quantité d'hydrogène nécessaire à l'ensemble des réactions réalisées dans cette étape est ajustée en fonction de la composition de la coupe pour avoir avantageusement seulement un léger excès d'hydrogène par rapport à la stoechiométrie théorique.

Les conditions opératoires sont choisies de telle façon que les réactifs et les produits soient en phase liquide. Il peut cependant être avantageux de choisir un mode de fonctionnement tel que les produits soient partiellement vaporisés à la sortie du réacteur, ce qui facilite le contrôle thermique de la réaction. La température peut varier de 20 à 200 °C, de préférence de 50 à 150 °C ou mieux de 60 à 100 °C. La pression peut être ajustée entre 0,1 et 5 MPa, de préférence entre 0,5 et 4 MPa et avantageusement entre 0,5 et 3 MPa, de telle façon que les réactifs, au moins en partie, soient en phase liquide. La vitesse spatiale peut être comprise entre 0,5 et 20 h⁻¹ et de préférence entre 1 et 10 h⁻¹, avec un rapport H₂ / dioléfines (molaire) de 0,5 à 5 et de préférence de 1 à 3.

Le ou les réacteurs d'hydroisomérisation peuvent avantageusement être suivis d'une colonne de stabilisation qui élimine les traces d'hydrocarbures gazeux éventuellement présents dans l'hydrogène de charge.

L'objet de la deuxième étape (zone 2) est de séparer par distillation la coupe C₄ issue de l'étape précédente, amenée par un conduit 3, pour obtenir d'une part une fraction contenant l'isobutène et la majorité du butène-1, d'autre part une fraction contenant une faible quantité de butène-1, le butène-2 et le n-butane. L'isobutène ainsi concentré, récupéré par un conduit 5, peut être destiné à des usages variés. La fraction butène-2 est dirigée par un conduit 4 vers l'étape de métathèse.

La fraction butène-2 du fond de zone de distillation contient au plus 1 % et de préférence au plus 0,5 % en poids de butène-1, au plus 1 % et avantageusement au plus 0,5 % en poids cl'isobutène. Dès lors, la perte en butène-2 en tête de colonne, est avantageusement maintenue à au plus 3 % poids par rapport au butène-2 entrant dans la colonne. Une colonne de distillation optimisée fonctionne avec 90-120 plateaux et un taux de reflux/charge de 3-5.

La fraction butène-2 issue de l'étape précédente ne contient pas de polluant extérieur (par exemple polluants oxygénés venant d'une étape d'étherification) et peut donc être envoyée directement dans la troisième étape du procédé (zone 3). Dans cette dernière étape, le butène-2 est mis en réaction avec de l'éthylène amené par un conduit 6, pour donner du propylène par métathèse (sortant par un conduit 7). En raison de la faible quantité de butène-1 et d'isobutène dans la charge, la formation de sous-produits est très limitée.

La réaction de métathèse de l'éthylène avec le butène-2 peut être catalysée par des oxydes métalliques variés déposés sur des supports. On utilise de préférence un catalyseur comportant au moins un oxyde de rhénium déposé sur un support composé par un oxyde réfractaire contenant au moins de l'alumine, qui présente un caractère acide, comme par exemple l'alumine elle-même, les silice-alumines ou les zéolithes.

On peut citer à titre d'exemple préféré les catalyseurs comportant de l'heptoxyde de rhénium déposé sur une alumine gamma analogue à celle utilisée dans les catalyseurs de reforming, comme décrits dans le brevet US 4 795 734. La teneur en rhénium (exprimée en rhénium métallique) peut être comprise entre 0,01 et 20 %, de préférence entre 1 et 15 % en poids. Les catalyseurs sont soumis par exemple à une activation thermique finale à une température comprise entre 400 et 1000 °C pendant une durée de 10 minutes à 5 heures sous une atmosphère non-réductrice.

Les catalyseurs comportant de l'heptoxyde de rhénium déposé sur une alumine peuvent aussi être modifiés par l'adjonction d'un oxyde d'un autre métal. De tels catalyseurs modifiés comprennent par exemple du rhénium à l'état d'oxyde, de 0,01 à 20 % en poids exprimé en rhénium métallique, déposé sur un support contenant au moins 75 % en poids d'alumine et 0,01 à 30 % en poids d'au moins un oxyde d'un métal choisi dans le groupe formé par le niobium et le tantale, tel que décrit dans le brevet FR 2 709 125.

La réaction de métathèse est effectuée de préférence en phase liquide, en l'absence d'oxygène, de composés oxygénés et d'humidité, et à une température comprise entre 0 et 200 °C, de préférence entre 20 et 150 °C, sous une pression au moins égale à la tension de vapeur du mélange réactionnel à la température de réaction.

Le catalyseur peut être mis en oeuvre en lit fixe. Cependant, comme il doit être fréquemment régénéré, il est alors nécessaire de disposer d'au moins deux réacteurs en parallèle, l'un étant en fonctionnement pendant que l'autre est en régénération. On utilise de préférence un système de lit catalytique mobile comme il est décrit dans le brevet français FR 2 608 595. Le catalyseur est soutiré à intervalles de temps réguliers en bas du réacteur et transféré vers un système de régénération en continu, d'où il est renvoyé en haut du réacteur.

Compte tenu des limitations imposées par la thermodynamique, l'éthylène non converti est fractionné dans une première colonne de distillation et recyclé au réacteur de métathèse. Une deuxième colonne de distillation sépare le propylène et les hydrocarbures en C₄ non convertis qui peuvent être recyclés au réacteur de métathèse. Le schéma de fractionnement est donc plus simple que si une grande quantité de butène-1 avait été présente dans la charge, car il se serait alors formé davantage de pentènes et d'hexènes qu'il aurait fallu éliminer avant de recycler les butènes.

Lorsque le procédé est appliqué à une coupe C₄ de vapocraquage, il peut être avantageux d'intégrer l'unité de métathèse avec le craqueur, de manière à bénéficier du train de fractionnement de celui-ci.

L'exemple suivant illustre l'invention sans en limiter la portée.

### EXEMPLE 1

Une coupe C₄ en sortie de vapocraqueur présente la composition indiquée dans le tableau 1 (flux 1). Abréviations du tableau : MAPD = méthyl-acétylène + propadiène, BBV = butadiène-1,2 + butyne-1 + vinyl-acétylène.

Cette coupe C₄ est d'abord soumise à un traitement d'hydrogénation et d'hydroisomérisation. Elle est introduite en continu, avec le débit massique indiqué dans le tableau 1, et sous une pression de 2 MPa, dans un premier réacteur comprenant un lit fixe de 2,6 T d'un catalyseur constitué par du palladium sur alumine sulfuré au préalable. De l'hydrogène (mélangé à du méthane) est également injecté dans ce réacteur, comme indiqué dans le tableau 1 (flux 2). L'effluent de ce premier réacteur est ensuite traité dans un

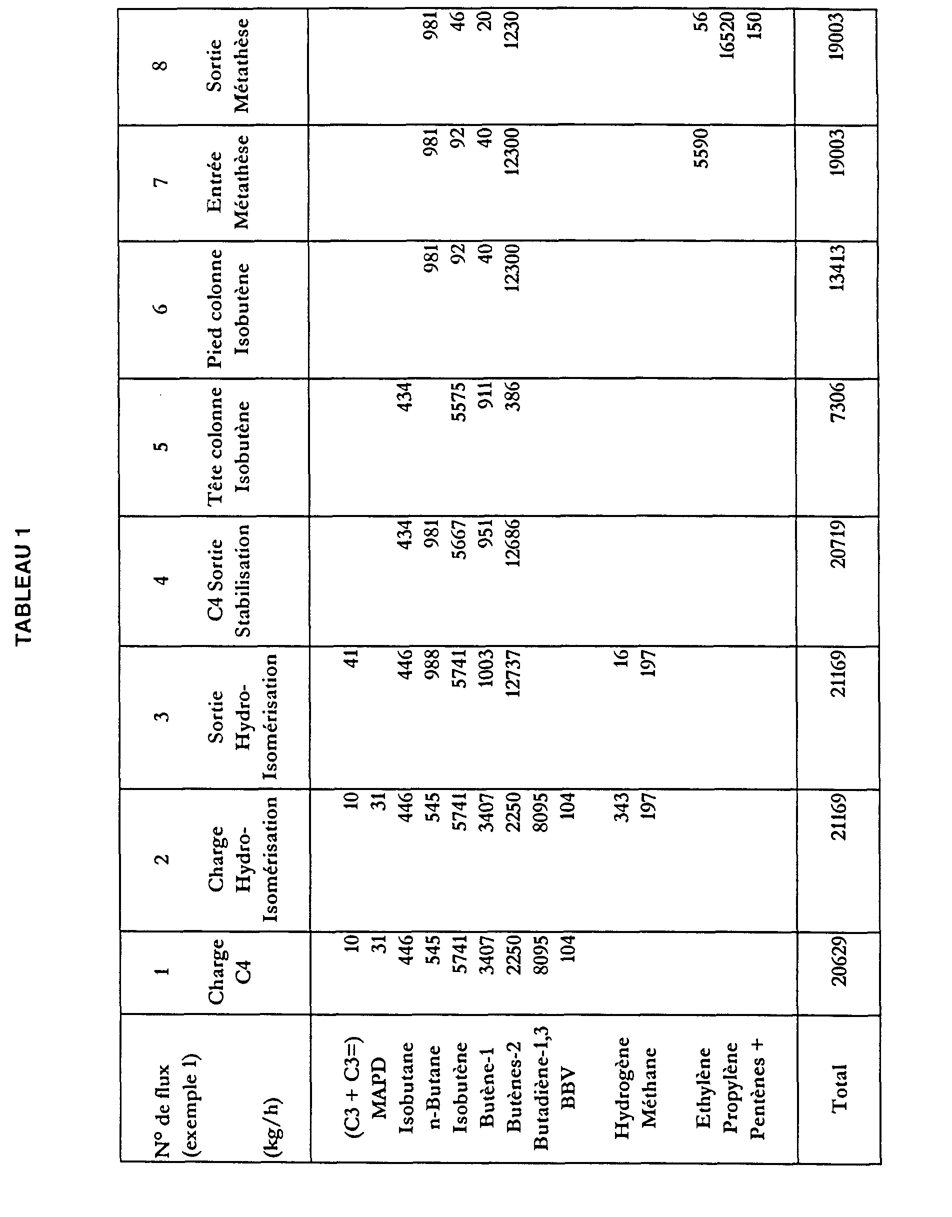

réacteur de finition chargé avec 2,5 T du même catalyseur. A la sortie (tableau 1, flux 3), la coupe est débarrassée des composés acétyléniques, et le butadiène a été transformé essentiellement en butènes, qui sont en majorité des butènes-2, le butène-1 ayant été isomérisé. La coupe est alors traitée dans une colonne de stabilisation, où l'hydrogène résiduel et le méthane sont séparés. Après ce traitement, la coupe a la composition du flux 4 (tableau 1).

Dans la deuxième étape, la coupe C₄ hydroisomérisée est soumise à un fractionnement dans une colonne de distillation. Cette colonne comporte environ 90 plateaux et fonctionne à une pression de 0,7 MPa au ballon de reflux, de façon à permettre l'utilisation d'eau de réfrigération dans le condenseur de tête. Le taux de reflux est ajusté pour d'une part limiter la perte en butène-2 dans le distillat à environ 3 %, et d'autre part réduire les teneurs en butène-1 et en isobutène dans le produit de fond respectivement à 0,3 % et 0,7 %, afin de limiter au maximum la formation des sous-produits pentènes et hexènes dans l'étape ultérieure de métathèse.

Dans la troisième étape, la fraction de pied de distillation qui contient principalement du butène-2 est mise en réaction avec de l'éthylène (composition globale : flux 7 du tableau 1) sur un catalyseur de métathèse, constitué par de l'oxyde de rhénium sur alumine gamma (8 % en poids en rhénium métal), préparé selon les enseignements du brevet US 4 795 734. La coupe C₄ est mélangée à l'entrée du réacteur de métathèse avec de l'éthylène d'appoint, ainsi qu'avec des flux de recyclage d'éthylène et de butènes. Ce réacteur fonctionne en lit mobile, comme décrit dans le brevet FR 2 608 595, à une température de 35 °C et sous une pression de 3,5 MPa, et il est couplé avec un régénérateur fonctionnant à 550 °C sous pression atmosphérique. Le catalyseur est soutiré à intervalles de temps réguliers en bas du réacteur et transféré vers le régénérateur, d'où il est renvoyé en haut du réacteur, les transferts se faisant à travers des sas tampons. En sortie de réacteur, l'éthylène non converti est fractionné dans une première colonne de distillation et recyclé. Une deuxième colonne de distillation sépare le propylène et les hydrocarbures en C₄ non convertis qui sont également recyclés. La composition de l'effluent de métathèse est indiquée dans le tableau 1, flux 8.

Le bilan global de la transformation s'établit donc comme suit. Pour 100 parties en poids (pp) de coupe C₄ sortie du vapocraqueur, on consomme 1,6 pp d'hydrogène et 28 pp d'éthylène, et on produit 27 pp d'isobutène et 83 pp de propylène. Au niveau du vapocraqueur d'où est issue la coupe C₄ traitée, ce bilan représente donc une faible consommation d'éthylène, qui permet une production supplémentaire de propylène importante, et ceci sans avoir à modifier les conditions de marche du craqueur.

L'avantage de ce procédé est donc de produire de façon très sélective un propylène de qualité polymérisation, grâce notamment à la métathèse d'une charge de butène-2 ne contenant que de faibles quantités de butène-1 et d'isobutène, charge obtenue par isomérisation et fractionnement d'une coupe C₄.

## Revendications

1. Procédé de conversion d'une coupe C₄ oléfinique en isobutène et en propylène, ladite coupe contenant notamment des dioléfines, du butène-1, du butène-2, de l'isobutène et des impuretés acétyléniques, **caractérisé en ce que** ledit procédé comporte les étapes suivantes se déroulant successivement :
1) hydrogénation sélective des dioléfines et des impuretés acétyléniques avec isomérisation du butène-1 en butène-2, de façon à obtenir un effluent contenant majoritairement du butène-2 et de l'isobutène, et ne contenant pratiquement pas de dioléfines ni de composés acétyléniques,
2) séparation par distillation d'une coupe de tête contenant majoritairement l'isobutène et le butène-1 non converti dans la première étape, et d'une coupe de pied contenant essentiellement le butène-2 et le butane, au plus 1 % en poids d'isobutène et au plus 1 % en poids de butène-1,
3) métathèse de la coupe butène-2 issue de l'étape précédente avec de l'éthylène, en présence d'un catalyseur comportant au moins un oxyde de rhénium déposé sur un support, à une température comprise entre 0 et 100 °C, et à une pression au moins égale à la tension de vapeur du mélange réactionnel à la température de réaction, de façon à obtenir un effluent contenant du propylène, la métathèse étant suivie d'une séparation du propylène.

2. Procédé selon la revendication 1, **caractérisé en ce que** l'étape 1 a lieu par passage de ladite coupe en phase liquide sur un catalyseur comprenant au moins un métal choisi dans le groupe formé par le nickel, le palladium et le platine, déposé sur un support, à une température de 20-200 °C, une pression de 0,1-5 MPa, une vitesse spatiale de 0,5-10 h⁻¹, avec un rapport H₂ / dioléfines (molaire) de 0,5 à 5 et de préférence de 1 à 3.

3. Procédé selon la revendication 2, **caractérisé en ce que** le catalyseur contient 0,05-10 % en poids de soufre.

4. Procédé selon la revendication 2, **caractérisé en ce que** le catalyseur de l'étape d'hydrogénation sélective a été traité, avant son chargement dans le réacteur d'hydrogénation, par au moins un composé soufré dilué dans un solvant, et que le catalyseur obtenu contenant 0,05 à 10 % de soufre (en poids) est chargé dans le réacteur et activé sous atmosphère neutre ou réductrice à une température comprise entre 20 et 300 °C, une pression comprise entre 0,1 et 5 MPa et une VVH comprise entre 50 et 600 h⁻¹, et que la charge est mise au contact dudit catalyseur activé.

5. Procédé selon l'une des revendications 2 à 4, **caractérisé en ce que** le catalyseur d'hydrogénation sélective est constitué par du palladium déposé sur de l'alumine, et du soufre.

6. Procédé selon la revendication 1, **caractérisé en ce que** la métathèse a lieu en présence d'un catalyseur contenant de l'oxyde de rhénium à raison de 0,01 à 20 % en poids exprimé en rhénium métallique, déposé sur un support contenant au moins 75 % en poids d'alumine et 0,01 à 30 % en poids d'au moins un oxyde d'un métal choisi dans le groupe formé par le niobium et le tantale.

7. Procédé selon l'une des revendications 1 ou 6, **caractérisé en ce que** la métathèse est effectuée avec un catalyseur en lit mobile.

8. Procédé selon la revendication 1, **caractérisé en ce que** la coupe de tête de l'étape 2 contient au plus 3 % en poids de butène-2.

9. Procédé selon l'une des revendications précédentes, **caractérisé en ce que** la coupe de tête est soumise à une polymérisation de l'isobutène.

10. Procédé selon l'une des revendications précédentes, **caractérisé en ce que** l'hydrogénation et l'isomérisation ont lieu simultanément dans l'étape 1).

11. Procédé selon l'une des revendications précédentes, **caractérisé en ce que** la coupe C4 est choisie parmi les coupes C4 issues du vapocraquage et les coupes C4 issues du craquage catalytique.

12. Installation pour la conversion d'une coupe C₄ oléfinique en isobutène et en propylène, **caractérisée en ce qu'**elle comporte successivement :
1) une zone 1 d'hydrogénation sélective avec isomérisation du butène-1 en butène-2, ladite zone comportant au moins un moyen 1 pour l'introduction de la coupe à convertir, au moins un moyen 3 pour la sortie de l'effluent et au moins un moyen 2 pour l'introduction de l'hydrogène, ladite zone comportant également au moins un lit de catalyseur,
2) une zone 2 de séparation, comportant au moins un moyen 3 pour l'introduction de l'effluent issu de la zone 1, au moins un moyen 5 pour la sortie de l'isobutène et du butène-1, au moins un moyen 4 pour la sortie du butène-2 et du n-butane,
3) une zone 3 de métathèse contenant au moins un catalyseur à base d'oxyde de rhénium déposé sur un support, et comprenant au moins un moyen 4 d'introduction de l'effluent issu de la zone 2, au moins un moyen 6 d'introduction de l'éthylène et au moins un moyen 7 pour la sortie du propylène.

13. Installation selon la revendication 12, **caractérisée en ce que** la zone de métathèse contient un lit mobile de catalyseur.

14. Installation selon la revendication 12, **caractérisée en ce que** le catalyseur de la zone 1 comprend au moins un métal choisi dans le groupe formé par le nickel, le palladium et le platine, déposé sur un support.

15. Installation selon l'une des revendications 12 à 14, **caractérisée en ce que** le moyen d'introduction de la coupe C₄ à convertir est relié à une zone de vapocraquage, et **en ce que** le moyen d'introduction de l'éthylène dans la zone de métathèse est relié à la zone de vapocraquage.

## Claims

1. A process for the conversion of an olefinic C₄ cut to isobutene and propylene, the cut containing diolefins, butene-1, butene-2, isobutene and acetylenic impurities, **characterized in that** said process comprises the following successive steps:
1) selective hydrogenation of diolefins and acetylenic impurities with isomerisation of butene-1 to butene-2 to obtain an effluent containing mainly butene-2 and isobutene, and containing practically no diolefins or acetylenic compounds,;
2) separating, by distillation, an overhead cut containing mainly isobutene and butene-1 which was not converted during the first step, and a bottom cut containing essentially butene-2 and butane, at most 1% by weight of isobutene and at most 1% by weight of butene-1;
3) metathesis of the butene-2 cut from the preceding step with ethylene, in the presence of a catalyst comprising at least one rhenium oxide deposited on a support, at a temperature in the range 0°C to 100°C, and at a pressure which is at least equal to the vapour tension of the reaction mixture at the reaction temperature, to obtain an effluent containing propylene, metathesis being followed by separation of the propylene.

2. A process according to claim 1, **characterized in that** step 1 is carried out by passing said cut in the liquid phase over a catalyst comprising at least one metal selected from the group formed by nickel, palladium and platinum, deposited on a support, at a temperature of 20-200°C, a pressure of 0.1-5 MPa, a space velocity of 0.5-10 h⁻¹, with an H₂/diolefin (molar) ratio of 0.5 to 5, preferably 1 to 3.

3. A process according to claim 2, **characterized in that** the catalyst contains 0.05% to 10% by weight of sulphur.

4. A process according to claim 2, **characterized in that** before charging it into the hydrogenation reactor, the catalyst for the hydrogenation step is treated with at least one sulphur-containing compound diluted in a solvent, then the catalyst obtained containing 0.05% to 10% (by weight) of sulphur is charged into the reactor and activated in a neutral or reducing atmosphere at a temperature which is in the range 20°C to 300°C, a pressure which is in the range 0.1 MPa to 5 MPa and a HSV which is in the range 50 h⁻¹ to 600 h⁻¹, and the feed is brought into contact with said activated catalyst.

5. A process according to any one of claims 2 to 4, **characterized in that** the selective hydrogenation catalyst is constituted by palladium deposited on alumina, and sulphur.

6. A process according to claim 1, **characterized in that** metathesis takes place in the presence of a catalyst containing rhenium oxide in a proportion of 0.01% to 20% by weight expressed as metallic rhenium, deposited on a support containing at least 75% by weight of alumina and 0.01% to 30% by weight of at least one oxide of a metal selected from the group formed by niobium and tantalum.

7. A process according to any one of claims 1 to 6, **characterized in that** metathesis is carried out using a catalyst in a moving bed.

8. A process according to claim 1, **characterized in that** the overhead cut from step 2 contains at most 3% by weight of butene-2.

9. A process according to any one of the preceding claims, **characterized in that** the overhead cut undergoes isobutene polymerisation.

10. A process according to any one of the preceding claims, **characterized in that** hydrogenation and isomerization are carried out simultaneously in step 1).

11. A process according to any one of the preceding claims, **characterized in that** the C4 cut is selected among C4 cuts obtained by steam-cracking and C4 cuts obtained by catalytic cracking.

12. An installation for converting an olefinic C₄ cut to isobutene and propylene, **characterized in that** it comprises, in succession:
1) a zone 1 for selective hydrogenation with isomerisation of butene-1 to butene-2, said zone comprising at least one means 1 for introducing a cut to be converted, at least one means 3 for removing effluent and at least one means 2 for introducing hydrogen, said zone also comprising at least one bed of a catalyst;
2) a separation zone 2, comprising at least one means 3 for introducing effluent from zone 1, at least one means 5 for removing isobutene and butene-1, and at least one means 4 for removing butene-2 and n-butane;
3) a metathesis zone 3 containing at least one catalyst, based on a rhenium oxide deposited on a support, and comprising at least one means 4 for introducing effluent from zone 2, at least one means 6 for introducing ethylene and at least one means 7 for removing propylene.

13. An installation according to claim 12, **characterized in that** the metathesis zone contains a moving catalyst bed.

14. An installation according to claim 12, **characterized in that** the catalyst of zone 1 comprises at least one metal selected from the group formed by nickel, palladium and platinum, deposited on a support.

15. An installation according to any one of claims 12 to 14, **characterized in that** the means for introducing the C₄ cut to be converted is connected to a steam cracking zone, and **in that** the nitrogen for introducing ethylene into the metathesis zone is connected to the steam cracking zone.

## Patentansprüche

1. Verfahren zur Umwandlung eines olefinischen C₄-Schnitts zu Isobuten und Propylen, wobei der Schnitt insbesondere die Diolefine, 1-Buten, 2-Buten, Isobuten und acetylenische Verunreinigungen enthält, **dadurch gekennzeichnet, dass** das Verfahren die folgenden aufeinanderfolgend ablaufenden Stufen aufweist:
1) selektive Hydrierung der Diolefine und der acetylenischen Verunreinigungen mit Isomerisierung des 1-Butens zu 2-Buten in einer Weise, um einen Abstrom zu erhalten, der hauptsächlich 2-Buten und Isobuten enthält und praktisch weder Diolefine noch acetylenische Verbindungen enthält,
2) Abtrennung durch Destillation von einem Kopfschnitt, der hauptsächlich Isobuten und 1-Buten enthält, das in der ersten Stufe nicht umgewandelt ist und von einem Bodenschnitt, der im wesentlichen 2-Buten und Butan, höchstens 1 Gew.-% Isobuten und höchstens 1 Gew.-% 1-Buten enthält,
3) Metathesis des 2-Buten-Schnitts aus der vorhergehenden Stufe mit Ethylen in Gegenwart eines Katalysators, der wenigstens ein Rheniumoxid, abgeschieden auf einen Träger, bei einer Temperatur, die zwischen 0 und 100 °C liegt und bei einem Druck, der wenigstens gleich der Dampfspannung der Reaktionsmischung bei der Reaktionstemperatur ist, aufweist, derart, dass ein Abstrom erhalten wird, der Propylen enthält, wobei die Metathesis von einer Trennung des Propylens gefolgt ist.

2. Verfahren nach Anspruch 1, **dadurch gekennzeichnet, dass** die Stufe 1 durch Passieren dieses Schnitts in flüssiger Phase über einen Katalysator stattfindet, der wenigstens ein Metall umfasst, das gewählt ist aus der Gruppe, die gebildet ist durch Nickel, Palladium und Platin, abgeschieden auf einem Träger, bei einer Temperatur von 20-200 °C, unter einem Druck von 0,1-5 MPa, bei einer Raumgeschwindigkeit von 0,5-10 h⁻¹ mit einem H₂/Diolefin-Verhältnis (molar) von 0,5 bis 5 und vorzugsweise von 1 bis 3.

3. Verfahren nach Anspruch 2, **dadurch gekennzeichnet, dass** der Katalysator 0,05-10 Gew.-% Schwefel enthält.

4. Verfahren nach Anspruch 3, **dadurch gekennzeichnet, dass** der Katalysator der selektiven Hydrierstufe vor dessen Ladung in den Hydrierreaktor durch wenigstens eine in einem Lösungsmittel verdünnte schwefelhaltige Verbindung behandelt worden ist und dass der erhaltene Katalysator, der 0,5 bis 10% Schwefel (in Gewicht) enthält, in den Reaktor geladen wird und unter neutraler oder reduzierender Atmosphäre bei einer Temperatur, die zwischen 20 und 300 °C liegt, einem Druck, der zwischen 0,1 und 5 MPa liegt und einer VVH, die zwischen 50 und 600 h⁻¹ liegt, aktiviert wird und dass die Charge mit diesem aktivierten Katalysator in Kontakt gebracht wird.

5. Verfahren nach einem der Ansprüche 2 bis 4, **dadurch gekennzeichnet, dass** der Katalysator zur selektiven Hydrierung durch Palladium abgeschieden auf Aluminiumoxid und durch Schwefel gebildet ist.

6. Verfahren nach Anspruch 1, **dadurch gekennzeichnet, dass** die Metathesis in Gegenwart eines Katalysators stattfindet, der Rheniumoxid im Verhältnis von 0,01 bis 20 Gew.-%, ausgedrückt als metallisches Rhenium, abgelagert auf einem Träger enthält, der wenigstens 75 Gew.-% Aluminiumoxid und 0,01 bis 30 Gew.-% wenigstens eines Oxids eines Metalls enthält, das aus der Gruppe gewählt ist, die gebildet wird durch Niob und Tantal.

7. Verfahren nach einem der Ansprüche 1 oder 6, **dadurch gekennzeichnet, dass** die Metathesis mit einem Katalysator im beweglichen Bett durchgeführt wird.

8. Verfahren nach Anspruch 1, **dadurch gekennzeichnet, dass** der Kopfschnitt der Stufe 2 höchstens 3 Gew.-% 2-Buten enthält.

9. Verfahren nach einem der vorhergehenden Ansprüche, **dadurch gekennzeichnet, dass** der Kopfschnitt einer Polymerisation des Isobutens unterzogen wird.

10. Verfahren nach einem der vorhergehenden Ansprüche, **dadurch gekennzeichnet, dass** die Hydrierung und Isomerisierung gleichzeitig in der Stufe 1 stattfinden.

11. Verfahren nach einem der vorhergehenden Ansprüche, **dadurch gekennzeichnet, dass** der C₄-Schnitt unter den C₄-Schnitten aus dem Dampfcracken und den C₄-Schnitten aus dem katalytischen Cracken gewählt ist.

12. Anlage für die Umwandlung eines olefinischen C₄-Schnitts zu Isobuten und Propylen, **dadurch gekennzeichnet, dass** sie aufeinanderfolgend aufweist:
1) eine Zone 1 zur selektiven Hydrierung unter Isomerisierung des 1-Butens zu 2-Buten, wobei diese Zone wenigstens ein Mittel 1 zur Einführung des umzuwandelnden Schnitts, wenigstens ein Mittel 3 zum Austritt des Abstroms und wenigstens ein Mittel 2 zur Einführung von Wasserstoff umfasst, wobei diese Zone gleichermaßen wenigstens ein Katalysatorbett umfasst,
2) eine Zone 2 zur Trennung, die wenigstens ein Mittel 3 zur Einführung des Abstroms aus der Zone 1, wenigstens ein Mittel 5 für den Austritt des Isobutens und des 1-Butens, wenigstens ein Mittel 4 für den Austritt vom 2-Buten und n-Butan umfasst,
3) eine Zone 3 zur Metathesis, die wenigstens einen Katalysator auf Basis von Rheniumoxid, abgeschieden auf einen Träger, enthält und wenigstens ein Mittel 4 zur Einführung des Abstroms aus der Zone 2, wenigstens ein Mittel 6 zur Einführung des Ethylens und wenigstens ein Mittel 7 zum Austritt des Proplyens umfasst.

13. Anlage nach Anspruch 12, **dadurch gekennzeichnet, dass** die Metathesiszone ein bewegliches Katalysatorbett enthält.

14. Anlage nach Anspruch 12, **dadurch gekennzeichnet, dass** der Katalysator der Zone 1 wenigstens ein Metall umfasst, das gewählt ist aus der Gruppe, die gebildet ist durch Nickel, Palladium und Platin, abgeschieden auf einem Träger.

15. Anlage nach einem der Ansprüche 12 bis 14, **dadurch gekennzeichnet, dass** das Mittel zur Einführung des umzuwandelnden C₄-Schnitts mit einer Dampfcrackzone verbunden ist und dadurch, dass das Mittel zu Einführung des Ethylens in die Metathesiszone mit der Dampfcrackzone verbunden ist.
